(19) Europäisches Patentamt
European Patent Office
Office européen des brevets

(11) **EP 2 505 162 B1**

(12) **EUROPEAN PATENT SPECIFICATION**

(45) Date of publication and mention of the grant of the patent:
**01.11.2017 Bulletin 2017/44**

(51) Int Cl.:
***A61B 34/10*** *(2016.01)*     ***A61B 90/00*** *(2016.01)*
***G06T 7/38*** *(2017.01)*

(21) Application number: **12161756.7**

(22) Date of filing: **28.03.2012**

(54) **Method and apparatus for generating medical image of body organ by using 3-D model**

Verfahren und Vorrichtung zum Erzeugen eines medizinischen Bildes eines Körperorgans unter Verwendung eines 3-D-Modells

Procédé et appareil de génération d'images médicales d'organes corporels en utilisant un modèle 3-D

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.03.2011 US 201161468754 P**
            **29.08.2011 KR 20110086694**

(43) Date of publication of application:
**03.10.2012 Bulletin 2012/40**

(73) Proprietor: **Samsung Electronics Co., Ltd.**
**Suwon-si, Gyeonggi-do, 443-742 (KR)**

(72) Inventors:
 • **Kim, Jung-bae**
    **Gyeonggi-do (KR)**
 • **Bang, Won-chul**
    **Gyeonggi-do (KR)**
 • **Hwang, Young-kyoo**
    **Gyeonggi-do (KR)**
 • **Kim, Yong-sun**
    **Gyeonggi-do (KR)**

(74) Representative: **Grünecker Patent- und**
**Rechtsanwälte**
**PartG mbB**
**Leopoldstraße 4**
**80802 München (DE)**

(56) References cited:
**EP-A1- 2 277 441**      **WO-A1-01/20552**
**US-A1- 2008 269 602**    **US-A1- 2009 163 799**
**US-A1- 2011 028 843**    **US-B1- 6 423 009**

**Description**

BACKGROUND

1. Field

The present disclosure relates to a method and apparatus for generating a medical image of a body organ by using a three-dimensional (3-D) model.

2. Description of the Related Art

In traditional methods of diagnosing and treating diseases, a state of a disease is confirmed with the naked eye after performing a laparotomy and then an incision or plastic surgery is performed on a lesion part with the use of large surgical instruments. However, recently, a method of treating diseases without making an incision in the body of a patient has been developed since it has becomes possible to obtain high resolution medical images and also to minutely control a medical instrument due to the progress of medical technology. In this method, treatment of a disease is performed while directly inserting a catheter or a medical needle into a blood vessel or a body part after making a small hole in the skin of the patient and then observing the inside of the body of the patient by using a medical imaging apparatus. This method is referred to as a "surgical operation using image", an "interventional image-based surgical operation", or a "mediate image-based surgical operation". A surgeon determines a location of an internal organ or lesion through images. Moreover, the surgeon needs to be aware of any change due to the patient's respiration or movement during a surgical operation. Thus, the surgeon needs to accurately and rapidly determine the patient's respiration or movement based on real time images to perform the surgical operation, but it is not easy to determine a shape of the internal organ or lesion with the naked eye. Thus, in order to solve this problem, methods and apparatuses for allowing the surgeon to determine a shape and location of an internal organ in real time have been developed. US2008/269602 A1, WO01/20552 A1, US6423009 B1, US2011/028843, US2009/163799 and EP2277441 A1 disclose devices and methods related to the invention.

SUMMARY

Provided are methods of generating the right image for fast and accurate tracing of an internal organ in a real time medical image of a patient.
Provided are apparatuses of generating the right image for fast and accurate tracing of an internal organ in a real time medical image of a patient.
Provided are computer-readable recording mediums having recorded thereon a program for executing the method.
Additional aspects will be set forth in part in the description which follows and, in part, will be apparent from the description, or may be learned by practice of the presented embodiments.
According to an aspect of the present invention, a method of generating an image of an organ includes: generating a three-dimensional (3-D) model of at least one organ of a patient, based on a medical image of the at least one organ; generating a plurality of matched images by matching a plurality of images, which show a change of a shape of the at least one organ due to a body activity of the patient, and the 3-D model of the at least one organ; and selecting one of the plurality of matched images based on the current body condition of the patient and outputting a selected matched image.
The invention is defined by the appended claims.
Preferred embodiments are disclosed in the dependent claims.

BRIEF DESCRIPTION OF THE DRAWINGS

These and/or other aspects will become apparent and more readily appreciated from the following description of the embodiments, taken in conjunction with the accompanying drawings in which:

FIG. 1 is a diagram illustrating a configuration of a system for generating an image of a body organ, according to an embodiment of the present invention;
FIG. 2 is a block diagram illustrating a configuration of an image matching device of FIG. 1;
FIG. 3 is a diagram illustrating a process of extracting location coordinate information of a boundary and an internal structure of an organ;
FIG. 4 is a flowchart illustrating a process in which an image matching unit fits a private model, in which a transformation of an organ is reflected, to a location of the organ in an ultrasound image for each image.

FIG. 5 illustrates a process of obtaining an affine transformation function in a two-dimensional (2-D) image;

FIG. 6 illustrates a process of matching an image performed by an image matching unit;

FIG. 7 is a graph illustrating an up and down movement of an absolute location of a diaphragm; and

FIG. 8 is a flowchart illustrating a method of tracing a dynamic organ and a lesion based on a three-dimensional (3-D) organ model.

DETAILED DESCRIPTION

Exemplary embodiments of the present invention will now be described more fully with reference to the accompanying drawings. In the following description, well-known functions or constructions are not be described in detail if it is determined that they would obscure the invention due to unnecessary detail.

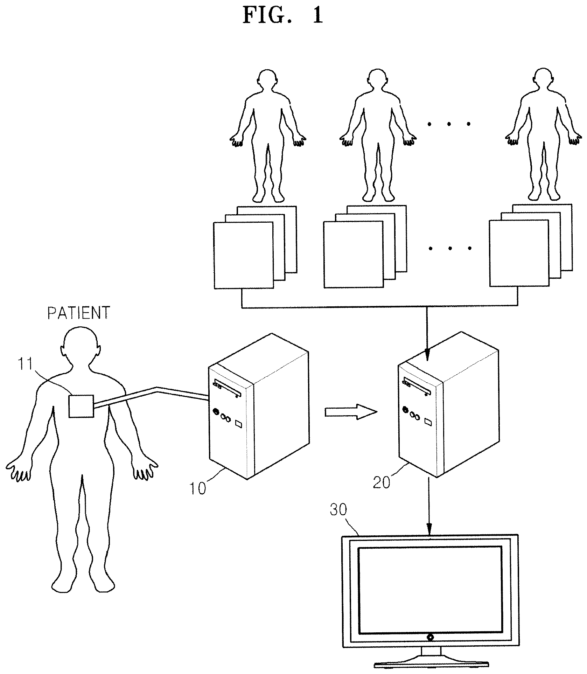

FIG. 1 is a diagram illustrating a configuration of a system for generating an image of a body organ, according to an embodiment of the present invention. Referring to FIG. 1, the system includes an image detection device 10, an image matching device 20, and an image display device 30. The image detection device 10 generates image data by using a response that is generated by transmitting a source signal generated from a probe 11 installed therein to a target part of a patient's body. The source signal may be a signal such as an ultrasound signal, an X-ray, or the like. As an example, a case where the image detection device 10 is an ultrasonography machine which captures three-dimensional (3-D) images of the patient's body by using ultrasound is explained below.

In the ultrasonography machine, the probe 11 is generally in the form of a piezoelectric transducer. If an ultrasound signal in the range of 2 to 18 MHz is transmitted from the probe 11 of the image detection device 10 to a part of the inside of the patient's body, this ultrasound signal is partially reflected from strata between various different tissues. In particular, the ultrasound is reflected from parts of which densities are changed in the inside of the body, for example, blood cells of blood plasma, small structures of organs, and the like. The reflected ultrasound vibrates the piezoelectric transducer of the probe 11, and the piezoelectric transducer outputs electrical pulses due to the vibration. The electrical pulses are converted into images.

The image detection device 10 may output two-dimensional (2-D) images and may also output 3-D images. A method in which the image detection device 10 outputs 3-D images is as follows. The image detection device 10 captures a plurality of cross-sectional images of a part of the patient's body while changing a location and orientation of the probe 11 over the patient's body. In addition, the image detection device 10 accumulates the cross-sectional images and thereby generates 3-D volume image data indicating three-dimensionally the part of the patient's body. In this manner, a method of generating the 3-D volume image data by accumulating the cross-sectional images is referred to as multi-planar reconstruction (MPR) method.

However, images which may be obtained by the image detection device 10, for example, ultrasound images, may be obtained in real time, but it is difficult to clearly identify an outline and internal structure of an organ or a lesion through the ultrasound images.

In computed tomography (CT) images or magnetic resonance (MR) images, a location of an organ or a location of a lesion may be clearly identified. However, when the patient breathes or moves during a surgical operation, the shape of the organ may be transformed or the location of the organ may be changed, and an image reflecting the real time change may be not obtained by using the CT images or MR images. That is, the CT images may not be output in real time because the CT images are obtained by using radiation and thus require short time photographing due to a danger to the patient or surgeon of prolonged radiation exposure. The MR images may not be output in real time because it takes a long time to capture them.

Thus, it is necessary to provide a method and apparatus which may capture images in real time and also clearly identify an outline and internal structure of an organ or a lesion. Thus, embodiments which will be explained below provide a method in which a location or transformation of an organ or lesion may be correctly identified by outputting images in which images detected in real time are matched to a model of an organ or a model of a lesion.

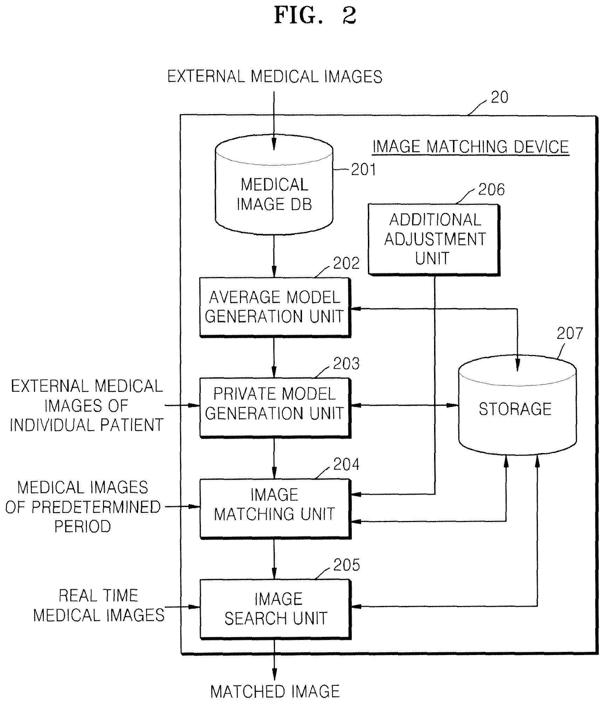

FIG. 2 is a block diagram illustrating a configuration of the image matching device 20 of FIG. 1. Referring to FIG. 2, the image matching device 20 includes a medical image database (DB) 201, an average model generation unit 202, a private model generation unit 203, an image matching unit 204, an image search unit 205, an additional adjustment unit 206, and a storage 207.

The average model generation unit 202 generates an average model of an organ by receiving various medical images of a patient and then processing them. In the current embodiment of the present invention, an organ of a patient is traced by using a private model, i.e., a personalized model of the patient. The average model is generated by the average model generation unit 202 as a preparatory step for generating the private model. This is because, since characteristics of an organ, such as shape and size, are different for each individual person, it is necessary to reflect the characteristics of each individual to provide an accurate surgical operation environment. Various pieces of image information of each individual may be used to obtain an accurate average model. In addition, images at various points of breathing may be obtained to reflect a form of an organ which is changed according to the breathing.

In detail, the average model generation unit 202 receives images (hereinafter, referred to as "external medical images"), which a medical expert has captured for diagnosis of a patient, directly from a photographing apparatus or from an image storage medium. Thus, it is desirable to receive images, which make it possible to easily analyze outlines of an organ or a lesion or characteristics of the inside of the organ, as the external medical images. For example, CT images or MR images may be input as the external medical images.

The external medical images may be stored in the medical image DB 201, and the average model generation unit 202 may receive the external medical images stored in the medical image DB 201. The medical image DB 201 may store medical images of various individuals, which may be captured by the photographing apparatus or may be input from the image storage medium. When receiving the external medical images from the medical image DB 201, the average model generation unit 202 may receive all or some of the external medical images from the medical image DB 201 depending on a selection of a user.

The average model generation unit 202 may apply a 3-D active shape model (ASM) algorithm based on the received external medical images. In order to apply the 3-D ASM algorithm, the average model generation unit 202 extracts shape, size, and anatomic features of an organ from the received external medical images by analyzing the received external medical images, and generates an average model of the organ by averaging them. The 3-D ASM algorithm is disclosed in the paper "The Use of Active Shape Models For Locating Structure in Medical Images" published in 1994 by T.F.Cootes, A.Hill, C.J.Taylor and J.Haslam. It is possible to obtain an average shape of the organ by applying the 3-D ASM algorithm, and the average shape of the organ may be transformed by modifying variables.

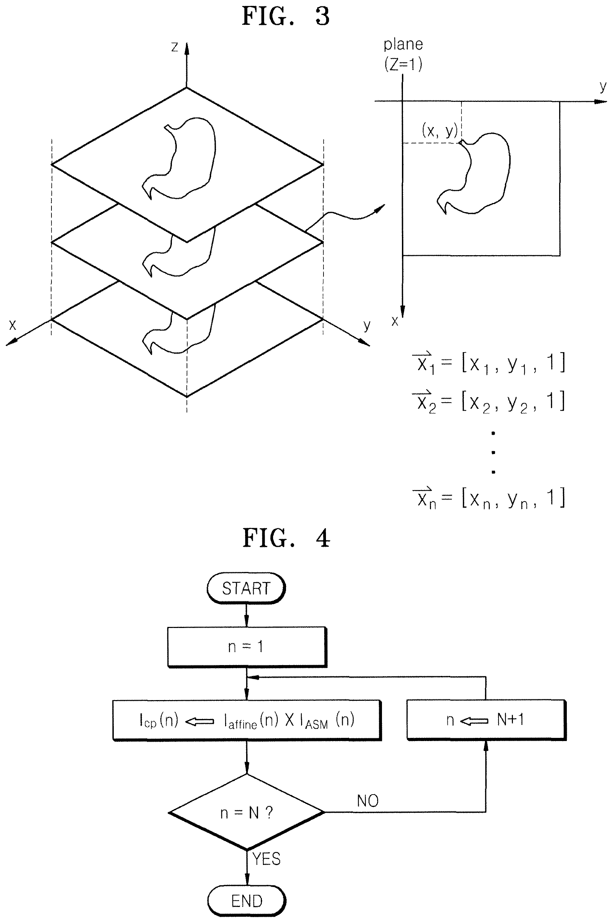

FIG. 3 is a diagram for explaining a process of analyzing the external medical images, which is performed by the average model generation unit 202. FIG. 4 roughly illustrates a process of extracting location coordinate information of a boundary and internal structure of an organ from the external medical images, for example, the CT or MR images. When the external medical images are input to the average model generation unit 202, the average model generation unit 202 performs an operation of extracting the location coordinate information of the boundary and internal structure of the organ by using different methods depending on whether the external medical images are 2-D images or 3-D images. For example, an internal structure of a liver may include a hepatic artery, a hepatic vein, and a hepatic duct and boundaries between them.

If 2-D images are input as the external medical images, the average model generation unit 202 obtains 3-D volume images indicating three-dimensionally a target part by accumulating a plurality of cross-sectional images to generate a 3-D model. This method of obtaining the 3-D volume images is illustrated in the left side of FIG. 3. In more detail, before accumulating the plurality of cross-sectional images, the location coordinate information of the boundary and internal structure of the organ is extracted from each of the plurality of cross-sectional images. Then, it is possible to obtain 3-D coordinate information by adding coordinate information of an axis of direction, in which the plurality of cross-sectional images are accumulated, to the extracted information. For example, since the image illustrated in the right of FIG. 3 is an image whose value of the Z-axis is 1, location coordinate value of a boundary extracted from the image is always 1. That is, 3-D coordinate information of the image illustrated in the right of FIG. 3 is [X,Y,1]. Thus, since coordinate information of cross-sections of images illustrated in the left of FIG. 3 is 2-D coordinate information [X,Y], both a coordinate value of the Z-axis and the 2-D coordinate information [X,Y] are extracted to obtain the location coordinate information of the images illustrated in the left of FIG. 3. Then, the location coordinate information of the images will be 3-D coordinate information [X,Y,Z]. If 3-D images are input as the external medical images, cross-sections of the 3-D images are extracted at predetermined intervals and then the same process as the case where 2-D images are input as the external medical images are performed, thereby obtaining 3-D location coordinate information. In this process, location coordinate information of a boundary of an organ in 2-D images may be automatically or semi-automatically obtained by using an algorithm, and may also be manually input by a user with reference to output image information. For example, in a method of automatically obtaining the location coordinate information of the boundary of the organ, it is possible to obtain location coordinate information of a part in which brightness of an image is abruptly changed, and also it is possible to extract a location of which a frequency value is largest, as a boundary location by using a discrete time Fourier transform (DTFT). In a method of semi-automatically obtaining the location coordinate information of the boundary of the organ, if information about a boundary point of an image is input by a user, it is possible to extract the location coordinate of a boundary based on the boundary point, similar to the method of automatically obtaining the location coordinate information. Since the boundary of the organ is continuous and has a looped curve shape, it is possible to obtain information about the whole boundary of the organ by using this characteristic. Since the method of semi-automatically obtaining the location coordinate information does not require searching for the whole of an image, it is possible to rapidly obtain a result compared to the method of automatically obtaining the location coordinate information. In a method of manually obtaining the location coordinate information of the boundary of the organ, a user may directly designate coordinates of a boundary while viewing the image. At this time, since an interval at which the coordinates of the boundary is designated may not be continuous, it is possible to continuously extract the boundary by performing interpolation with respect to discontinuous sections. If the location coordinate information of the organ or a lesion, obtained by using the above methods, is output after setting a brightness value of a voxel corresponding to the location coordinate to a predetermined value, the user may confirm shapes of the organ or the lesion expressed three-dimensionally and graphically. For example, if a brightness value of boundary coordinates of a target organ is set to a minimum value, namely the darkest value, an image of the target organ will have a dark form in an output image. If the brightness value of the target organ is set to a medium value between a white color and a black color and the brightness value of a lesion is set to the black color, it is possible to easily distinguish the lesion from the target organ with the naked eye. The location coordinate information of boundaries and internal structures of a plurality of organs, obtained by using the above methods, may be defined as a data set and be used to perform the 3-D ASM algorithm. The 3-D ASM algorithm is explained below.

In order to apply the 3-D ASM algorithm, coordinate axes of location coordinates of the boundaries and internal structures of the plurality of organs are fit to each other. Fitting the coordinate axes to each other means fitting the centers of gravities of the plurality of organs to one origin and aligning directions of the plurality of. Thereafter, landmark points are determined in the location coordinate information of the boundaries and internal structures of the plurality of organs. The landmark points are basic points used to apply the 3-D ASM algorithm. The landmark points are determined by using following method.

First, points in which a characteristic of a target is distinctly reflected are determined as the landmark points. For example, the points may include division points of blood vessels of a liver, a boundary between the right atrium and the left atrium in a heart, a boundary between a main vein and an outer wall of the heart, and the like.

Second, the highest points or the lowest points of a target in a predetermined coordinate system are determined as the landmark points.

Third, points for interpolating between the first determined points and the second determined points are determined as the landmark points along a boundary at predetermined intervals.

The determined landmark points may be represented by using coordinates of the X and Y axes in two dimensions, and may be represented by using coordinates of the X, Y, and Z axes in three dimensions. Thus, if coordinates of each of the landmark points are indicated as vectors $x_0, x_1, \cdots x_{n-1}$ in three dimensions (here, n is the number of landmark points), the vectors $x_0, x_1, \cdots x_{n-1}$ may be represented by the following equation 1.

$$x_{i0} = \left[ x_{i0}, y_{i0}, z_{i0} \right]$$
$$x_{i1} = \left[ x_{i1}, y_{i1}, z_{i1} \right]$$
$$\vdots$$
$$x_{in-1} = \left[ x_{in-1}, y_{in-1}, z_{in-1} \right] \quad (1)$$

The subscript i indicates location coordinate information of a boundary and internal structure of an organ, obtained in an i-th image. The number of the location coordinate information may be increased in some cases, and thus, the location coordinate information may be represented as a single vector to facilitate a calculation thereof. Then, a landmark point vector which expresses all the landmark points with a single vector may be defined by the following equation 2.

$$x_i = \left[ x_{i0}, y_{i0}, z_{i0}, x_{i1}, y_{i1}, z_{i1}, \cdots, x_{in-1}, y_{in-1}, z_{in-1} \right]^T \quad (2)$$

The size of the vector $x_i$ is $3n \times 1$. If the number of the data set is N, an average of the landmark points for all the data set may be represented as the following equation 3.

$$\bar{x} = \frac{1}{N} \sum_{i=1}^{N} x_i \tag{3}$$

Similarly, the size of the vector $\bar{x}$ is $3n \times 1$. The average model generation unit 202 obtains the average $\bar{x}$ of the landmark points by calculating equation 3, and, if a model is generated based on the average $\bar{x}$ of the landmark points, the model may become an average organ model. The 3-D ASM algorithm may not only generate the average organ model, but may change a form of the average organ model only by adjusting a plurality of parameters. Thus, the average model generation unit 202 calculates not only the average organ model but also an equation so that the plurality of parameters may be applied. Below, an equation for applying the plurality of parameters is explained.

By the following equation 4, the average $\bar{x}$ of the landmark points and differences between data may be represented. In equation 4, the subscript i indicates an i-th image. Thus, equation 4 indicates a difference between the landmark points of each image and the average of all images.

$$dx_i = x_i - \bar{x} \tag{4}$$

By using the difference, a covariance matrix for three variables x, y, and z may be defined as the following equation 5. The reason for obtaining the covariance matrix is to obtain a unit eigen-vector for the plurality of parameters to apply the 3-D ASM algorithm.

$$S = \frac{1}{N} \sum_{i=1}^{N} dx_i dx_i^T \quad (\exists 7| \quad 3n \times 3n\,) \tag{5}$$

If the unit eigen-vector of the covariance matrix S is $p_k$, the vector $p_k$ indicates a change of a model generated by using the 3-D ASM algorithm. For example, if a parameter $b_1$ multiplied by a vector $p_1$ is changed within the range of $-2\sqrt{\lambda_1} \leq b_1 < 2\sqrt{\lambda_1}$, a width of the model may be changed. If a parameter $b_2$ multiplied by a vector $p_2$ is changed within the range of $-2\sqrt{\lambda_2} \leq b_2 < 2\sqrt{\lambda_2}$, a height of the model may be changed. The unit eigen-vector $p_k$ of which a size is $3n \times 1$ may be obtained by using equation 6 as follows.

$$Sp_k = \lambda_k p_k \tag{6}$$

$\lambda_k$ indicates an eigen-value. Finally, the landmark point vector $\bar{x}$ to which the change of the model is applied may be calculated by using the average vector $\bar{x}$ of the landmark points as in the following equation 7.

$$x = \bar{x} + Pb \tag{7}$$

$p = (p_1, p_2, \cdots p_t)$ indicates t eigen-vectors (here, the size of the $p_k$ is $3n \times 1$, and the size of p is $3n \times t$.), $b = (b_1, b_2, \cdots b_t)^T$ indicates a weight of each eigen-vector (here, the size of the b is $t \times 1$).

The average model generation unit 202 may calculate $\bar{x}$ (the size thereof is $3n \times 1$), which indicates a form of an average organ model, and the vector $p = (p_1, p_2, \cdots p_t)$ $p_t$ (the size thereof is $3n \times t$), which is used to apply the change of the model by using the 3-D ASM algorithm, by using the equations.

The private model generation unit 203 receives the average organ model $\bar{x}$ and the vector $p = (p_1, p_2, \cdots p_t)$ from the average model generation unit 202 and then generates a private model through parameter processing of the 3-D ASM algorithm. Since shapes and sizes of organs of patients are different according to the individual patient, accuracy may be lowered if the average organ model is used as it is. For example, an organ of a patient may have a longer, wider, thicker, or thinner form compared to organs of other patients. In addition, if an organ of a patient includes a lesion, the private model generation unit 203 may include a location of the lesion to a model of the organ to accurately capture a shape and location of the lesion. Thus, the private model generation unit 203 receives external medical images of the individual patient from an external image photographing apparatus or a storage medium 207, analyzes a shape, size, and location of an organ of the individual patient, and, if there is a lesion, analyzes a shape, size, and location of the lesion. Below, this operation of the private model generation unit 203 is explained.

The private model generation unit 203 determines weights (the vector b) of eigen-vectors of the 3-D ASM algorithm for the individual patient, based on the medical images such as the CT or MR images in which a shape, size, and location of an organ may be clearly captured. Thus, first, the private model generation unit 203 receives the external medical images of the individual patient and obtains location coordinate information of a boundary and internal structure of an organ. At this time, in order to obtain the location coordinate information of the boundary and internal structure of the organ, the private model generation unit 203 uses the process of FIG. 3, namely the process of analyzing the external medical images, which is performed by the average model generation unit 202. Furthermore, by determining coordinate information of the landmark points through a method which is the same as that used when applying the 3-D ASM algorithm, it is possible to obtain the vector $\bar{x}$ (the size thereof is $3n \times 1$) which is a private landmark point set of the individual patient. An organ model generated based on the vector $\bar{x}$ may be a private model. If a characteristic $\left( p_k^T p_k = 1 \right)$ of a reversed function and unit eigen-vector is used in equation 7, the following equation 8 may be obtained. A value of $b = (b_1, b_2, \cdots b_t)^T$ is determined by equation 8.

$$b = P^T \left( x - \bar{x} \right) \qquad (8)$$

The vectors $\bar{x}$ and $p$ determined by the average model generation unit 202 may be stored in the storage 207 as a database of an average model for a target organ, and may be repeatedly used if necessary. In addition, the external medical images of the individual patient, input to the private model generation unit 202, may be additionally used when determining the average model stored in the database during a medical examination and treatment of another patient.

When the image matching unit 204 receives the vectors $x, \bar{x}, p, b$ from the private model generation unit 203, the image matching unit 204 may match the vectors with a patient's medical images received during a predetermined period. This matching means that a model using the 3-D ASM algorithm is overlapped with a location of an organ in an ultrasound medical image to output an output image. In detail, the matching means that it is possible to replace or overlap pixel or voxel values corresponding to coordinate information of a model formed by the 3-D ASM algorithm with a predetermined brightness. If the replacement operation is performed, an organ part is removed from an original ultrasound medical image and only a private model is output. If the overlap operation is performed, an image in which the original ultrasound medical image is overlapped with the private model, may be output. The overlapped image may be easily identified with the naked eye by differentiating a color thereof from that of another image. For example, it may be easy to identify a graphic figure with the naked eye by overlapping a private model with a black and white ultrasound image by using a blue color.

The medical images may be images captured in real time and, for example, may be the ultrasound images. The medical images may be 2-D or 3-D images. The predetermined period may be one breathing cycle. This is because a change of an organ also is generated during a breathing cycle of the body. For example, if one breathing cycle of a patient is 5 seconds, ultrasound images having 100 frames may be generated when ultrasound images are generated 20 frames per 1 second.

A process of matching, which is performed in the image matching unit 204, may be divided into two operations. The two operations include an operation of reflecting a change of an organ due to breathing to a 3-D organ model in ultrasound images input during a predetermined period and an operation of aligning the changed 3-D organ model to a target organ in the ultrasound images by performing scale control, axis rotation, and axis movement.

The operation of reflecting a change of an organ due to breathing to a 3-D organ model is as follows. Before matching the ultrasound images with medical images, a value of the vector b which is a weight of a parameter of the 3-D ASM algorithm is controlled by obtaining a location and change of an organ for each frame of the ultrasound images. A value of the vector b determined at this time does not have a large difference from a value of the vector b determined in the average model generation unit 202. This is because only a change due to the breathing is reflected in the image matching unit 204 and this change due to the breathing is smaller compared to changes in other individuals. Thus, when determining the value of the vector b, a modification is performed within a predetermined limited range based on the value of the vector b determined in the average model generation unit 202. In addition, a vector b of a previous frame may be reflected in a determination of a vector b of a next frame. This is because there is no large change during a short period between frames since a change of an organ during the breathing is continuous. If the value of the vector b is determined, it is possible to generate a private model, for each frame, in which a modification of an organ is reflected in each ultrasound image by using a calculation of the 3-D ASM algorithm.

FIG. 4 is a flowchart illustrating a process in which the image matching unit 204 fits a private model, in which a transformation of an organ is reflected for each image, to a location of an organ in an ultrasound image through rotation, scale control, and parallel displacement. In detail, FIG. 4 is a flowchart illustrating a process of performing one-to-one affine registration for each frame when the vector b, which is a weight value of an eigen-vector for each frame, is determined. If the number of frames is N and n is a frame number, a one-to-one match is performed from when n is 1 to when n becomes N. An affine transformation function is obtained by performing an iterative closest point (ICP) algorithm for each frame by using a landmark point set of an ultrasound image and a landmark point set of a model, and a 3-D body organ model image is obtained by using the affine transformation function. The ICP algorithm is an algorithm for rotating and parallel displacing other images and controlling scales of the other images based on an image to align a target in a plurality of images. The ICP algorithm is disclosed in detail in "Iterative point matching for registration of free-form curves and surfaces" by Zhengyou Zhang.

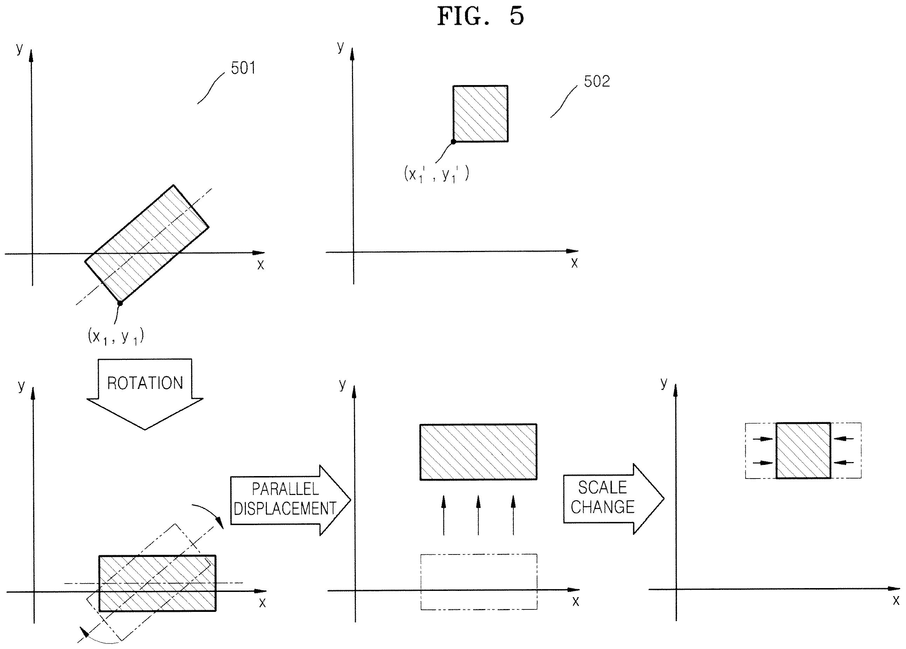

FIG. 5 illustrates a process of obtaining the affine transformation function in a 2-D image. A diagram 501 illustrates a state before applying the affine transformation, and a diagram 502 illustrates a state after applying the affine transformation. Although the rotation, the parallel displacement, and the scale control should be performed to apply the transformation, it is possible to determine coefficients of a matrix $T_{affine}$ of the affine transformation function by obtaining first coordinates and last coordinates through the following equation 9 in consideration that the affine transformation uses one to one point correspondence.

$$\begin{bmatrix} x_1' \\ y_1' \end{bmatrix} = T_{affine} \begin{bmatrix} x_1 \\ y_1 \\ 1 \end{bmatrix} = \begin{bmatrix} a_1 & b_1 & c_1 \\ a_2 & b_2 & c_2 \end{bmatrix} \begin{bmatrix} x_1 \\ y_1 \\ 1 \end{bmatrix} \tag{9}$$

Equation 10 is an equation for applying an affine transformation function obtained in three-dimensions to each frame.

$$x_{ICP}(n) = T_{affine}(n) \times x_{ASM}(n) \tag{10}$$

Here, n is an integer indicating an n-th frame ($1 \leq n \leq N$). $x_{ASM}(n)$ indicates a landmark point vector in which the vector b that is the weight value is changed in the image matching unit 204. $x_{ICP}(n)$ includes location coordinate information of organ boundaries and internal structures in which a modification is reflected for each frame. It is possible to confirm a graphic figure of an organ with the naked eye if a voxel value corresponding to location coordinates is replaced or overlapped with a predetermined brightness value in an ulrasonic image when matching the location coordinate information with the ultrasound image.

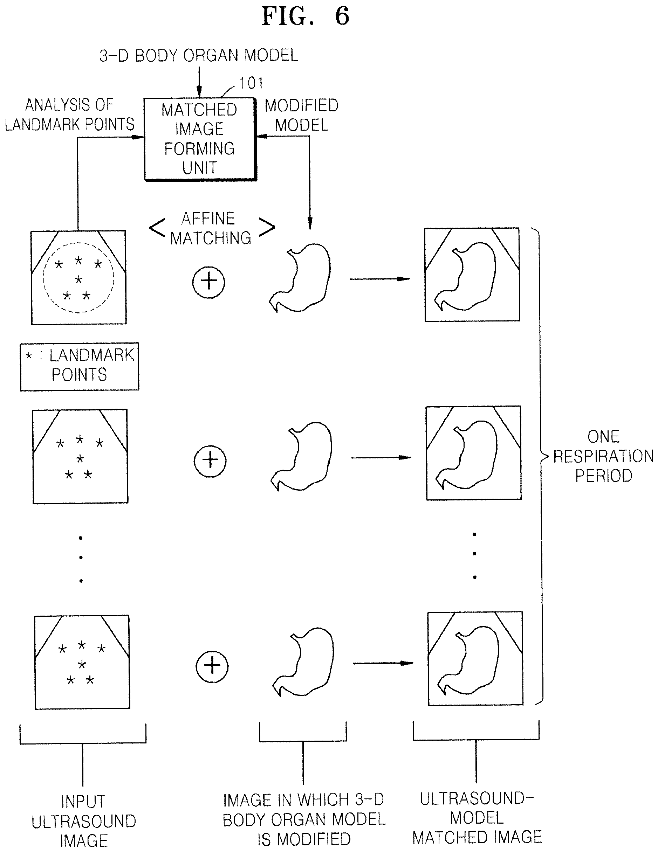

FIG. 6 illustrates a process of matching an image in the image matching unit 204. FIG. 6 illustrate a process in which the image matching unit 204 generates a matched image between a ultrasound image input during a predetermined period and a body organ model based on an ultrasound image input during one breathing cycle. In FIG. 6, the input ultrasound image is disposed in a left edge portion, and a mark * illustrated in the input ultrasound image indicates a landmark point. The input ultrasound image may reflect various forms of breathing from inspiration to expiration.

A private model generated in the private model generation unit 203 will be modified according to breathing. However, a modification according to the respiration will be smaller than that due to diversity between individuals. Thus, when reflecting a modification according to breathing, it may be faster and easier to adjust parameter values determined by the private model generation unit 203 compared to newly obtaining 3-D ASM algorithm. The affine transformation function $T_{affine}$ is applied through the ICP algorithm by using a landmark point in which the modification has been reflected and a landmark point of an organ of the ultrasound image. Through the affine transformation, a size and location of a 3-D organ model may be modified to be matched with a size and location of an organ of the ultrasound image. Combining a modified model with the ultrasound image may be performed through a method of replacing or overlapping a pixel or voxel value of the ultrasound image corresponding to a location of a model with a predetermined value. A matched image is referred to as an ultrasound-model matched image and may be stored in the storage 207.

The image search unit 205 performs processes of a surgical operation. In the surgical operation, a graphic shape of an organ is output in an ultrasound image, which is input in real time, on a screen, and then a surgeon performs the surgical operation while confirming the graphic shape of the organ with the naked eye. Detailed operations of this process are as follow. First, a real time medical image of a patient is received. At this time, the real time medical image may be an image which is the same as that received by the image matching unit 204. Thus, for example, if a real time ultrasound image is received, by comparing the real time ultrasound image with medical images input to the image matching unit 204 during a predetermined, an image which is most similar to the real time ultrasound image is determined, and an ultrasound-model matched image corresponding to the determined image is searched in the storage and then a searched ultrasound-model matched image is output.

As an example in which the image search unit 205 searches for a similar image in the ultrasound image, there is a method of determining an image by detecting a location of the diaphragm. If a location of the diaphragm is X in the real time ultrasound image, the method involves searching for an image having the smallest difference by calculating a difference between the location X and a location of each diaphragm in the medical images input to the image matching unit 204 during the predetermined period.

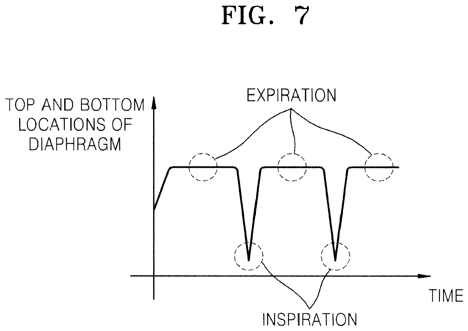

FIG. 7 is a graph illustrating an up and down movement of an absolute location of the diaphragm. On analyzing the graph, it is possible to confirm that the location of the diaphragm is regularly changed in a breathing cycle. A location of a probe 11 and a location of a patient may be fixed when capturing the medical images, which are input to the image matching unit 204 during the predetermined period, and the real time medical image, which are input to the image search unit 205. The reason is that a relative location of an organ in the image may be changed if the location of the probe 11 or the location of the patient is changed and it is not possible to accurately and rapidly perform a search operation when comparing images if the relative location is changed.

As another example in which the image search unit 205 searches for a similar image in the ultrasound image, there is a method of determining an image by using a brightness difference between pixels. That is, this method involves using the fact that a brightness difference between the most similar images is the smallest. In detail, when searching for an image similar to an image (a second image) of a frame of the real time medical image among the medical images (first images) input during the predetermined period to use for matching, a brightness difference between pixels of one of the first images and pixels of the second image is calculated and then a dispersion for the brightness difference is obtained. Next, brightness differences between pixels of the other images of the first images and pixels of the second image also are calculated and then dispersions for the brightness differences are obtained. Then, an image whose dispersion is the smallest may be determined as the most similar image.

The additional adjustment unit 206 may output an adjusted final result if a user adjusts the affine transformation function $T_{affine}$ and the parameters of the 3-D ASM algorithm while viewing an output image. That is, the user may perform accurate transformation while viewing the output image with the naked eye.

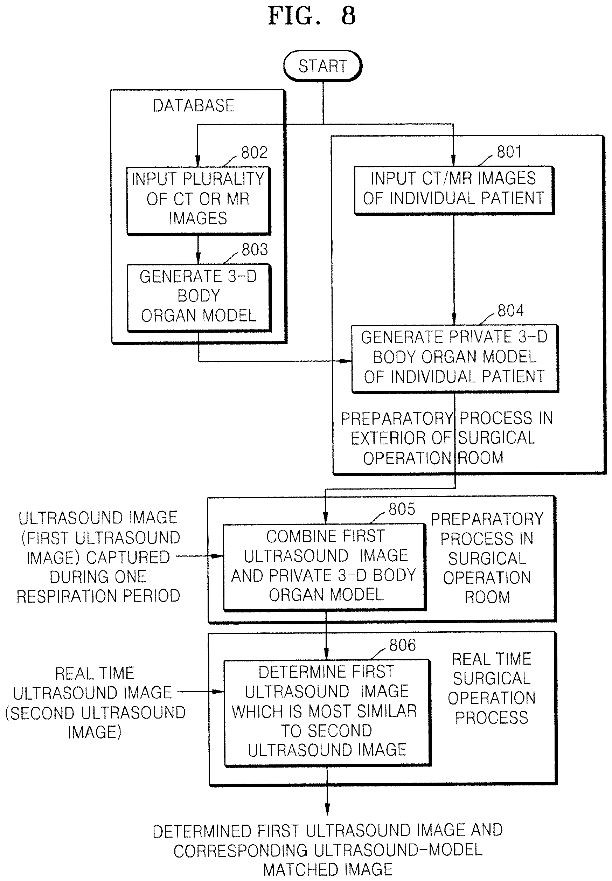

FIG. 8 is a flowchart illustrating a method of tracing a dynamic organ and a lesion based on a 3-D organ model. Results of operations 802 and 803 may be stored in the medical image database (DB) 201 of FIG. 2. In the operation 802, CT or MR images for various breathing cycles of individuals are received. In the operation 803, a 3-D body organ model is generated based on the received images. At this time, as stated above, the 3-D ASM algorithm may be used.

In operation 801, a CT or MR image of an individual patient is received. In operation 804, the 3-D body organ model generated in the operation 803 is modified based on the received image of the individual patient. A process of generating the modified 3-D body organ model, namely a private 3-D body organ model may be performed in the exterior of a surgical operation room as a preparatory process. In operation 805, ultrasound images (first ultrasound images) captured during one breathing cycle of a patient are received, and the first ultrasound images are matched with the private 3-D body organ model. A matched image is referred to as an ultrasound-model matched image, and may be stored in a temporary memory or in a storage medium such as storage. Operation 805 may be performed as a preparatory process in a surgical operation room. In operation 805, a location of the patient may be fixed. In addition, in operation 806, a location of a probe may be fixed. In operation 806, as a real operation in the surgical operation room, if an ultrasound image (a second ultrasound image) of the patient is input in real time, an image, which is most similar to the second ultrasound image, from among the first ultrasound images is determined and then an ultrasound-model matched image corresponding to the determined first ultrasound image is output.

The embodiments of the present general inventive concept can be written as computer programs and can be implemented in general-use digital computers that execute the programs using a computer readable recording medium.

Examples of the computer readable recording medium include magnetic storage media (e.g., ROM, floppy disks, hard disks, etc.), optical recording media (e.g., CD-ROMs, or DVDs), etc.

Even after all programs in a computer system including an operating system (OS) are deleted, a first program that is stored in a first storage unit in which a BIOS is stored, is not deleted. Thus, a user may install software automatically and may update software without any difficulty by invoking a second program for installing software automatically and updating software.

While this invention has been particularly shown and described with reference to preferred embodiments thereof, it will be understood by those of ordinary skill in the art that various changes in form and details may be made therein without departing from the scope of the invention as defined by the appended claims. The preferred embodiments should be considered in a descriptive sense only and not for purposes of limitation. Therefore, the scope of the invention is defined not by the detailed description of the invention but by the appended claims.

**Claims**

1.  A method of generating an image of an organ, the method comprising:

    generating a three-dimensional (3-D) model of at least one organ of a patient, based on medical images of the at least one organ;
    generating a plurality of matched images by matching a plurality of images, which show a change of a shape of the at least one organ due to a body activity of the patient, and the 3-D model of the at least one organ;
    receiving a real time medical image of the patient; and
    selecting one of the plurality of matched images based on the current body condition of the patient and outputting the selected matched image; **characterised in that** the selecting and outputting comprises selecting a matched image corresponding to an image, which is most similar to a real time medical image, from among the plurality of images.

2.  The method of claim 1, wherein the generating of the 3-D model comprises generating the 3-D model showing a shape of the at least one organ of the patient based on the medical image of the patient.

3.  The method of claim 1 or 2, wherein the generating of the plurality of matched images comprises:

    modifying the 3-D model based on the change of the shape of the at least one organ; and
    fitting a coordinate axis of the 3-D model to a coordinate axis of the plurality of images;
    in particular, wherein the generating of the plurality of matched images further comprises generating the plurality of matched images by overlapping pixels or voxels of the plurality of images with pixels or voxels of a predetermined brightness.

4.  The method of any one of claims 1 to 3, wherein the selecting and outputting comprises calculating a difference between a location of a diaphragm in the plurality of images and a location of a diaphragm in the real time medical image, and selecting an image having the smallest difference.

5.  The method of any one of claims 1 to 4, wherein the generating of the 3-D model comprises:

    extracting location coordinate information of a boundary and internal structure of the at least one organ from the medical images;
    designating coordinates of landmark points in the location coordinate information; and
    generating a statistical external appearance model.

6.  The method of claim 5, wherein the generating of the 3-D model further comprises changing the statistical external appearance model into a model reflecting a shape characteristic of the at least one organ of the patient;
    in particular, wherein the generating of the 3-D model comprises reflecting the shape characteristic of the at least one organ of the patient onto the medical images of the patients.

7.  The method of claim 5 or 6, wherein the extracting of the location coordinate information comprises determining a position, of which a change of a brightness value is maximum in the medical image, as the location coordinate information of the boundary and internal structure of the at least one organ;
    in particular, wherein the extracting of the location coordinate information comprises determining a position, of which a frequency value of a discrete time Fourier transform (DTFT) is maximum in the medical image, as the location coordinate information of the boundary and internal structure of the at least one organ.

8. The method of claim 5 or 6, wherein the extracting of the location coordinate information comprises determining the location coordinate information of the boundary and internal structure of the at least one organ based on coordinates input by a user.

9. The method of any one of claims 1 to 8, wherein the plurality of images are images captured at predetermined intervals in a breathing cycle of the patient.

10. The method of any one of claims 1 to 9, wherein the medical image of the at least one organ are images captured by using a computed tomography (CT) method; or
wherein the medical images of the at least one organ are images captured by using a magnetic resonance (MR) method.

11. The method of any one of claims 1 to 10, wherein the plurality of images and the real time image of the patient are ultrasound images.

12. The method of any one of claims 1 to 11, wherein, instead of the generating of the 3-D model, a pre-generated 3-D model stored in a database is received.

13. The method of claim 6, wherein the shape characteristic comprises a shape and location of a lesion.

14. An apparatus for generating an image of an organ, the apparatus comprising:

an organ model generation unit (202) for generating a 3-D model of at least one organ of a patient, based on medical images of the at least one organ;
an image matching unit (204) for generating a plurality of matched images by matching a plurality of images, which shows a change of a shape of the at least one organ due to a body activity of the patient, and the 3-D model of the at least one organ;
means for capturing a real time medical image of the patient; and
an image search unit (205) for selecting one of the plurality of matched images based on the current body condition of the patient;
charactered in that the image search unit selects a matched image corresponding to an image, which is most similar to a real time medical image, from among the plurality of images.

15. The apparatus of claim 21, further comprising an additional adjustment unit (206) for further adjusting the plurality of matched images, according to an input of a user.

16. A computer-readable recording medium having recorded thereon a program for executing the method of any one of claims claim 1 to 13 when executed on the apparatus of one of claims 14 or 15.

**Patentansprüche**

1. Ein Verfahren zum Erzeugen eines Bildes eines Organs, wobei das Verfahren umfasst:

Erzeugen eines dreidimensionalen (3-D) Modells von zumindest einem Organ eines Patienten auf der Grundlage medizinischer Bilder des zumindest einen Organs;
Erzeugen mehrerer passender Bilder durch Abgleichen mehrerer Bilder, die eine Änderung einer Form des zumindest einen Organs aufgrund einer Körperaktivität des Patienten zeigen, mit dem 3-D Modell des zumindest einen Organs;
Empfangen eines medizinischen Echtzeitbildes des Patienten; und
Auswählen von einem der mehreren passenden Bilder auf der Grundlage des gegenwärtigen Körperzustands des Patienten und Ausgeben des ausgewählten passenden Bildes; **dadurch gekennzeichnet, dass** das Auswählen und Ausgeben ein Auswählen eine passenden Bildes entsprechend einem Bild umfasst, das einem medizinischen Echtzeitbild am ähnlichsten ist.

**2.** Das Verfahren von Anspruch 1, in dem das Erzeugen des 3-D Modells ein Erzeugen des 3-D Modells, das eine Form des zumindest einen Organs des Patienten zeigt, auf der Grundlage des medizinischen Bildes des Patienten umfasst.

**3.** Das Verfahren von Anspruch 1 oder 2, in dem das Erzeugen der mehreren passenden Bilder umfasst:

Ändern des 3-D Modells auf der Grundlage der Änderung der Form des zumindest einen Organs; und
Anpassen einer Koordinatenachse des 3-D Modells an eine Koordinatenachse der mehreren Bilder;
wobei insbesondere das Erzeugen der mehreren passenden Bilder weiterhin ein Erzeugen der mehreren passenden Bilder durch Überlappen von Pixeln oder Voxeln der mehreren Bilder mit Pixeln oder Voxeln einer vorbestimmten Helligkeit umfasst.

**4.** Das Verfahren von einem der Ansprüche 1 bis 3, in dem das Auswählen und Ausgeben ein Berechnen eines Unterschieds zwischen einer Position eines Diaphragmas in den mehreren Bilder und einer Position eines Diaphragmas in dem medizinischen Echtzeitbild und Auswählen eines Bildes mit dem kleinsten Unterschied umfasst.

**5.** Das Verfahren von einem der Ansprüche 1 bis 4, in dem das Erzeugen des 3-D Modells umfasst:

Extrahieren von Positionskoordinateninformationen einer Grenze und einer inneren Struktur des zumindest einen Organs aus den medizinischen Bildern;
Kennzeichnen von Koordinaten von Landmark-Punkten in den Positionskoordinateninformationen; und
Erzeugen eines statistischen Modells eines äußeren Aussehens.

**6.** Das Verfahren von Anspruch 5, in dem das Erzeugen des 3-D Modells weiterhin ein Ändern des statistischen Modells eines äußeren Aussehens in ein Model umfasst, das eine Formeigenschaft des zumindest einen Organs des Patienten wiedergibt;
wobei insbesondere das Erzeugen des 3-D Modells ein Spiegeln der Formeigenschaft des zumindest einen Organs des Patienten auf die medizinischen Bilder der Patienten umfasst.

**7.** Das Verfahren von Anspruch 5 oder 6, in dem das Extrahieren der Positionskoordinateninformationen ein Bestimmen einer Position, an der eine Änderung in dem Helligkeitswert in dem medizinischen Bild maximal ist, als die Positionskoordinateninformationen der Grenze und inneren Struktur des zumindest einen Organs umfasst;
wobei insbesondere das Extrahieren der Positionskoordinateninformationen ein Bestimmen einer Position, an der ein Frequenzwert einer diskreten Fourier-Zeit-Transformation (DTFT) in dem medizinischen Bild maximal ist, als die Positionskoordinateninformationen der Grenze und inneren Struktur des zumindest einen Organs umfasst.

**8.** Das Verfahren von Anspruch 5 oder 6, in dem das Extrahieren der Positionskoordinateninformationen ein Bestimmen der Positionskoordinateninformationen der Grenze und inneren Struktur des zumindest einen Organs auf der Grundlage von von einem Nutzer eingegebenen Koordinaten umfasst.

**9.** Das Verfahren von einem der Ansprüche 1 bis 8, in dem die mehreren Bilder Bilder sind, die in vorbestimmten Intervallen eines Atemzyklus des Patienten aufgenommen werden.

**10.** Das Verfahren von einem der Ansprüche 1 bis 9, in dem das medizinische Bild des zumindest einen Organs Bilder sind, die unter Verwendung einen Computertomographie (CT) - Verfahrens aufgenommen werden; oder
in dem die medizinischen Bilder des zumindest einen Organs Bilder sind, die unter Verwendung eines Magnetresonanz (MR) - Verfahrens aufgenommen werden.

**11.** Das Verfahren von einem der Ansprüche 1 bis 10, in dem die mehreren Bilder und das Echtzeitbild des Patienten Ultraschallbilder sind.

**12.** Das Verfahren von einem der Ansprüche 1 bis 11, in dem anstelle des Erzeugens des 3-D Modells ein zuvor erzeugtes 3-D Modell, das in einer Datenbank gespeichert ist, empfangen wird.

**13.** Das Verfahren von Anspruch 6, in dem die Formeigenschaft eine Form und Position einer Läsion umfasst.

**14.** Eine Vorrichtung zum Erzeugen eines Bildes eines Organs, wobei die Vorrichtung umfasst:

eine Organmodellerzeugungseinheit (202) zum Erzeugen eines 3-D Modells von zumindest einem Organ eines Patienten auf der Grundlage medizinischer Bilder des zumindest einen Organs;

eine Bildabgleicheinheit (204) zum Erzeugen mehrerer passender Bilder durch Abgleichen mehrerer Bilder, die eine Änderung einer Form des zumindest einen Organs aufgrund einer Körperaktivität des Patienten zeigen, mit dem 3-D Modell des zumindest einen Organs;

Mittel zum Aufnehmen eines medizinischen Echtzeitbildes des Patienten; und

eine Bildsucheinheit (205) zum Auswählen von einem der mehreren passenden Bilder auf der Grundlage des gegenwärtigen Körperzustands des Patienten, **dadurch gekennzeichnet, dass** die Bildsucheinheit ein passendes Bild entsprechend einem Bild aus den mehreren Bildern auswählt, das einem medizinischen Echtzeitbild am ähnlichsten ist.

**15.** Die Vorrichtung von Anspruch 21, weiterhin eine Zusatzanpassungseinheit (206) zum weiteren Anpassen der mehreren passenden Bilder gemäß einer Eingabe eines Nutzers umfassend.

**16.** Ein computerlesbares Aufzeichnungsmedium, das darauf aufgezeichnet ein Programm zum Ausführen des Verfahrens eines der Ansprüche 1 bis 13, wenn es auf der Vorrichtung eines der Ansprüche 14 oder 15 ausgeführt wird, umfasst.

**Revendications**

**1.** Procédé de production d'une image d'un organe, le procédé comprenant :

la production d'un modèle tridimensionnel (3-D) d'au moins un organe d'un patient, sur la base des images médicales du au moins un organe ;

la production d'une pluralité d'images qui correspondent en faisant correspondre une pluralité des images, qui montrent un changement d'une forme du au moins un organe dû à une activité corporelle du patient, et le modèle 3-D du au moins un organe ;

la réception d'une image médicale en temps réel du patient ; et

la sélection de l'une de la pluralité des images qui correspondent sur la base de l'état corporel présent du patient et l'émission de l'image sélectionnée qui correspond; **caractérisée en ce que** la sélection et l'émission comprennent la sélection d'une image qui correspond correspondant à une image, qui est la plus semblable à une image médicale en temps réel, parmi la pluralité des images.

**2.** Procédé selon la revendication 1, dans lequel la production du modèle 3-D comprend la production du modèle 3-D montrant une forme du au moins un organe du patient sur la base de l'image médicale du patient.

**3.** Procédé selon la revendication 1 ou 2, dans lequel la production de la pluralité des images qui correspondent comprend :

la modification du modèle 3-D sur la base du changement de forme du au moins un organe ; et

l'ajustement d'un axe de coordonnées du modèle 3-D à un axe de coordonnées de la pluralité des images ;

en particulier, la production de la pluralité des images qui correspondent comprenant en outre la production de la pluralité des images qui correspondent par des pixels ou des voxels chevauchant de la pluralité des images avec des pixels ou des voxels d'une brillance prédéterminée.

**4.** Procédé selon l'une quelconque des revendications 1 à 3, dans lequel la sélection et l'émission comprennent le calcul d'une différence entre un emplacement d'un diaphragme dans la pluralité des images et un emplacement d'un diaphragme dans l'image médicale en temps réel, et la sélection d'une image ayant la plus petite différence.

**5.** Procédé selon l'une quelconque des revendications 1 à 4, dans lequel la production du modèle 3-D comprend :

l'extraction de l'information des coordonnées de localisation d'une limite et d'une structure interne du au moins un organe des images médicales ;

la désignation des coordonnées des points de repère dans l'information des coordonnées de localisation ; et

la production d'un modèle d'apparence externe statistique.

**6.** Procédé selon la revendication 5, dans lequel la production du modèle 3-D comprend en outre le changement du modèle d'apparence externe statistique en un modèle reflétant une forme caractéristique du au moins un organe du patient ;

en particulier, où la production du modèle 3-D comprend la réflexion de la forme caractéristique du au moins un organe du patient sur les images médicales des patients.

7. Procédé selon la revendication 5 ou 6, dans lequel l'extraction de l'information des coordonnées de localisation comprend la détermination d'une position, dont un changement d'une valeur de brillance est maximal dans l'image médicale, comme l'information des coordonnées de localisation de la limite et de la structure interne du au moins un organe ;

en particulier, où l'extraction de l'information des coordonnées de localisation comprend la détermination d'une position, dont une valeur de fréquence d'une transformée de Fourier à temps discret (DTFT) est maximale dans l'image médicale, comme l'information des coordonnées de localisation de la limite et de la structure interne du au moins un organe.

8. Procédé selon la revendication 5 ou 6, dans lequel l'extraction de l'information des coordonnées de localisation comprend la détermination de l'information des coordonnées de localisation de la limite et de la structure interne du au moins un organe sur la base des coordonnées saisies par un utilisateur.

9. Procédé selon l'une quelconque des revendications 1 à 8, dans lequel la pluralité des images sont des images capturées à des intervalles prédéterminés dans un cycle de respiration du patient.

10. Procédé selon l'une quelconque des revendications 1 à 9, dans lequel l'image médicale du au moins un organe sont des images capturées en utilisant un procédé de tomographie assistée par ordinateur (CT) ; ou

dans lequel les images médicales du au moins un organe sont des images capturées en utilisant un procédé de résonance magnétique (MR).

11. Procédé selon l'une quelconque des revendications 1 à 10, dans lequel la pluralité des images et l'image en temps réel du patient sont des images par ultrasons.

12. Procédé selon l'une quelconque des revendications 1 à 11, dans lequel, au lieu de la production d'un modèle 3-D, un modèle 3-D pré-généré stocké dans une base de données est reçu.

13. Procédé selon la revendication 6, dans lequel la forme caractéristique comprend une forme et une localisation d'une lésion.

14. Appareil de production d'une image d'un organe, l'appareil comprenant :

une unité de production d'un modèle d'organe (202) pour la production d'un modèle 3-D d'au moins un organe d'un patient, sur la base des images médicales du au moins un organe ;

une unité de correspondance d'image (204) pour produire une pluralité d'images qui correspondent en faisant correspondre une pluralité d'images, qui montre un changement d'une forme du au moins un organe dû à une activité corporelle du patient, et du modèle 3-D du au moins un organe ;

un moyen de capture d'une image médicale en temps réel du patient ; et

une unité de recherche d'image (205) pour sélectionner l'une de la pluralité des images qui correspondent sur la base de l'état corporel présent du patient ;

**caractérisé en ce que** l'unité de recherche d'image sélectionne une image qui correspond correspondant à une image, qui est la plus semblable à une image médicale en temps réel, parmi la pluralité des images.

15. Appareil selon la revendication 21, comprenant en outre une unité d'ajustement additionnel (206) pour l'ajustement de manière supplémentaire de la pluralité des images qui correspondent, selon une saisie d'un utilisateur.

16. Milieu d'enregistrement pouvant être lu par ordinateur présentant enregistré dessus un programme d'exécution du procédé selon l'une quelconque des revendications 1 à 13 lorsqu'exécuté sur l'appareil selon l'une des revendications 14 ou 15.

# FIG. 1

# FIG. 2

EXTERNAL MEDICAL IMAGES

20

IMAGE MATCHING DEVICE

MEDICAL
IMAGE DB
201

ADDITIONAL
ADJUSTMENT
UNIT
206

AVERAGE MODEL
GENERATION UNIT
202

STORAGE
207

EXTERNAL MEDICAL
IMAGES OF
INDIVIDUAL PATIENT

PRIVATE MODEL
GENERATION UNIT
203

MEDICAL IMAGES
OF PREDETERMINED
PERIOD

IMAGE
MATCHING UNIT
204

REAL TIME
MEDICAL IMAGES

IMAGE
SEARCH UNIT
205

MATCHED IMAGE

# FIG. 3

$$\vec{x}_1 = [x_1, y_1, 1]$$
$$\vec{x}_2 = [x_2, y_2, 1]$$
$$\vdots$$
$$\vec{x}_n = [x_n, y_n, 1]$$

# FIG. 4

# FIG. 5

EP 2 505 162 B1

# FIG. 6

# FIG. 7

# FIG. 8

START

DATABASE

INPUT PLURALITY
OF CT OR MR
IMAGES
802

GENERATE 3-D
BODY
ORGAN MODEL
803

INPUT CT/MR IMAGES
OF INDIVIDUAL PATIENT
801

GENERATE PRIVATE 3-D
BODY ORGAN MODEL
OF INDIVIDUAL PATIENT
804

PREPARATORY PROCESS IN
EXTERIOR OF SURGICAL
OPERATION ROOM

ULTRASOUND IMAGE
(FIRST ULTRASOUND
IMAGE) CAPTURED
DURING ONE
RESPIRATION PERIOD

COMBINE FIRST
ULTRASOUND IMAGE
AND PRIVATE 3-D BODY
ORGAN MODEL
805

PREPARATORY
PROCESS IN
SURGICAL
OPERATION
ROOM

REAL TIME
ULTRASOUND IMAGE
(SECOND ULTRASOUND
IMAGE)

DETERMINE FIRST
ULTRASOUND IMAGE
WHICH IS MOST SIMILAR
TO SECOND
ULTRASOUND IMAGE
806

REAL TIME
SURGICAL
OPERATION
PROCESS

DETERMINED FIRST ULTRASOUND IMAGE AND
CORRESPONDING ULTRASOUND-MODEL
MATCHED IMAGE

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2008269602 A1
- WO 0120552 A1
- US 6423009 B1
- US 2011028843 A
- US 2009163799 A
- EP 2277441 A1

**Non-patent literature cited in the description**

- **T.F.COOTES ; A.HILL ; C.J.TAYLOR.** The Use of Active Shape Models For Locating Structure in Medical Images. *J.Haslam,* 1994